# EUROPEAN PATENT APPLICATION

(11) **EP 3 034 045 A1**
(43) Date of publication of application: **22.06.2016**
(21) Application number: 15200381.0
(22) Date of filing: 16.12.2015
(51) Int. Cl.: A61F 2/66, A61F 2/78

(54) **PROSTHESIS WITH PROSTHETIC SPACER DEVICES**

(30) Priority: 18.12.2014 US 201462093867 P
(71) Applicant: Fountainhead, LLC, Shepherd, MT 59079 (US)
(72) Inventor: Kania, Bruce G., Shepherd, MT 59079 (US); Stewart, Frank M., Bozeman, MT 59715 (US); Colvin, James M., Hilliard, OH 43026 (US); Schroeder, Ryan M., Columbus, OH 43206 (US)
(74) Representative: Hutchinson, Thomas Owen

(57) **Abstract**

A prosthetic device (10) comprises a plurality of polymer units (18, 18a, 18b, 18c, 18d) attached to one or more leaf springs (12a). The polymer units (18, 18a, 18b, 18c, 18d) are spaced apart and define spaces (28) between the units such that there is no contact between any two polymer units (18, 18a, 18b, 18c, 18d) at maximum deflection of the one or more leaf springs (12a). The polymer units (18, 18a, 18b, 18c, 18d) may be impermeable to water and the spaces (28) may facilitate free flow of liquids out of the device (10). The plurality of polymer units may comprise a heel piece (18c) and a toe piece (18a). One or both of the heel piece (18c) and the toe piece (18a) may extend beneath the one or more leaf springs (12a) such that the one or more leaf springs (12a) do not contact the ground when the device (10) is used for ambulatory movement. The toe piece (18a) may extend further down than the heel piece (18c) and may extend beneath the one or more leaf springs (12a) such that extension of the toe piece (18a) provides increased flexing of the one or more leaf springs (12a).

## Description

This application claims priority to U.S. Patent Application No. 62/093,867, filed December 18, 2014, which is hereby incorporated by reference in its entirety.

The following disclosure relates to prosthesis systems and devices including prosthetic feet.

Most conventional prosthetic feet are primarily leaf spring systems. These typically are used with a foot shell which provides a cosmetic feature as well as a functional one, in that the shell fills in space within a shoe that would otherwise impact the usability of a leaf spring foot. Foam material can be used to continue the "space filling" role above the ankle of the prosthesis, but this technique is not particularly robust and the device deteriorates quite quickly.

Use of a leaf spring without a shoe is not practical, so a foot shell approach remains fundamental to prosthetic foot design. This approach also has some ability to provide ideal sizing, so that a small number of leaf spring models can service all foot sizes. However, foot shell prostheses have a number of drawbacks.

The disadvantages of the foot shell include the fact that they are typically noisy. They also collect debris and grit, which can degrade and damage the prosthetic device. It can be difficult to put boots or high shoes on a prosthetic foot because a leaf spring foot only articulates slightly, and with certain footwear, such as dress shoes or women's shoes, the user can't walk comfortably or appropriately.

In wet conditions, the foot shell can collect water. When water enters a foot shell and gets trapped, the user may need to lie on his or her back and hold the foot up to drain the water. Because it is difficult to put on boots or high shoes for outdoor activities, some individuals who have a prosthetic foot use regular light hiking type shoes and suffer the resulting wet feet. The moisture also tends to break down material interfacing between the leaf spring and the foot shell. Due to movement or friction or compression, all of which occur in a foot shell system, some energy is lost.

Another drawback is that the foot shells sometimes pop off of the leaf spring itself during usage. In addition, removal of a foot shell is sometimes necessary for clean-up of the foot shell interior, but removing the foot shell can be difficult, and when done improperly can break a leaf spring. The composite construction of the leaf spring makes working with it somewhat challenging, in that the composite material sometimes splinters and will readily penetrate skin. In the high volume deflection environment of a foot, foot shells ultimately fail, typically faster than the leaf springs.

Accordingly, there is a need for a new prosthetic foot design that is durable, practical, and modular so it can be easily sized for different individuals. There is also a need for a prosthetic foot that is not noisy and does not collect and retain water in wet conditions. Finally, there is a need for a prosthetic foot that is easy to work with, particularly one that is easy to remove and clean.

Various aspects of the invention are set forth in the appendant claims.

According to an aspect of the invention, there is provided a prosthetic device comprising: at least one leaf spring; and a plurality of polymer units attached to the leaf spring, the polymer units being spaced apart and defining spaces between the units such that contact between any two polymer units does not interfere with deflection of the one or more leaf springs.

Suitably, the spaces facilitate free flow of liquids out of the device.

Suitably, the polymer units are impermeable to water.

Suitably, the plurality of polymer units comprise a heel piece and a toe piece.

Suitably, one or both of the heel piece and the toe piece extend beneath the leaf spring such that, in use, the one or more leaf springs do not contact the ground when the device is used for ambulatory movement.

Suitably, the toe piece extends further down than the heel piece.

Suitably, the plurality of polymer units comprise a toe piece extending beneath the one or more leaf springs such that extension of the toe piece provides increased flexing of the one or more leaf springs.

Suitably, one of the plurality of polymer units defines a storage compartment therein.

The polymer units may vary in levels of hardness. Suitably, the plurality of polymer units comprise a heel piece and a toe piece, the hardness of the toe piece being higher than the hardness of the heel piece, thereby providing higher energy return and better toe-off from the toe piece.

The plurality of polymer units may include a shank unit. The hardness of the shank unit may be relatively lower than hardness of the heel piece.

One or more polymer units may define a plurality of hollow portions.

At least one polymer unit may comprise an outer shell and an inner filling. The outer shell may be denser than the inner filling.

The present disclosure, in its many embodiments, alleviates to a great extent the disadvantages of known prosthetic foot designs by providing new prosthetic foot systems and devices comprising one or more leaf springs with multiple polymer pieces bonded to the springs. The polymer pieces are spaced apart so they do not interfere with the flexibility of the springs, do not produce rubbing noise, and do not trap water. By designing material onto the top of a leaf spring, exemplary embodiments of a prosthetic foot can replace the functionality of a foot shell. This design will not experience movement or friction, and compression is designable.

Prosthetic systems and devices of the present disclosure advantageously have a modular construction. More particularly, polymer pieces or units can be provided in various sizes. In exemplary embodiments, the toe piece is supplied in a variety of sizes so that one standard device may be used for several shoe sizes by changing only the toe piece.

Other advantages and objects of the present disclosure include that the spacing between the polymer units dampens heel shock when walking, providing a greater level of comfort. The design is also very quiet because the polymer pieces can be bonded directly onto the leaf springs. In addition, the design will not trap water; water entering the shoe is free to drain and will not collect within the prosthetic device. The polymer units are made of a material that is easy to shape and work with. Because the polymer units cover most of the surface of the leaf springs, there is minimal potential for pebbles, grit, or other debris to contact the leaf spring or get caught between the leaf spring and the polymer units of the device. In exemplary embodiments, the leaf springs are positioned above the bottom of the footwear, extending the life of the leaf spring.

Exemplary embodiments can even provide advantages for amputees over non-amputees. As discussed in more detail herein, the polymer units can be of varying durometers. In one example, the shank unit can be made extremely compressible such that the bending effect of the leaf spring maximizes energy return at toe-off and maximizes for lack of ingress of debris into the prosthetic device.

Exemplary embodiments of a prosthetic device comprise at least one leaf spring and a plurality of polymer units attached to the leaf spring. The polymer units are spaced apart and define spaces between the units such that contact between any two polymer units does not interfere with deflection of the leaf spring, and in exemplary embodiments there is no contact between any two polymer units at maximum deflection of the leaf spring. In exemplary embodiments, the polymer units may be impermeable to water and the spaces may facilitate free flow of liquids out of the device.

In exemplary embodiments, the polymer units vary in levels of hardness. One of the plurality of polymer units may define a storage compartment therein. The one or more polymer units may define a plurality of hollow portions. In exemplary embodiments, the polymer units are shaped so as to fill the interior of a conventional shoe. In exemplary embodiments, at least one polymer unit comprises an outer shell and an inner filling. In exemplary embodiments, the outer shell is denser than the inner filling.

In exemplary embodiments, the plurality of polymer units may comprise a heel piece and a toe piece. One or both of the heel piece and the toe piece may extend beneath the leaf spring such that the leaf spring does not contact the ground when the device is used for ambulatory movement. The toe piece may extend further down than the heel piece and may extend beneath the leaf spring such that extension of the toe piece provides increased flexing of the leaf spring. In exemplary embodiments, the polymer units include a shank unit. The prosthetic device may further comprise a cosmetic cover sock covering the prosthetic device.

Accordingly, it is seen that prosthetic devices and systems are provided. The disclosed devices and systems comprising one or more leaf springs with multiple polymer pieces bonded to the springs. The polymer pieces are spaced apart so they do not interfere with the flexibility of the springs, do not produce rubbing noise, and do not have spaces in which water can be trapped. These and other features and advantages will be appreciated from review of the following detailed description, along with the accompanying figures in which like reference numbers refer to like parts throughout.

The above-mentioned features and objects of the present disclosure will become more apparent with reference to the following description taken in conjunction with the accompanying drawings wherein like reference numerals denote like elements and in which:
FIG. 1 is a side cross-sectional view of an exemplary embodiment of a prosthetic device in accordance with the present disclosure;
FIG. 2 is a side cross-sectional view of an exemplary embodiment of a prosthetic device in accordance with the present disclosure;
FIG. 3 is a side cross-sectional view of an exemplary embodiment of a prosthetic device in accordance with the present disclosure;
FIG. 4 is a side cross-sectional view of an exemplary embodiment of a prosthetic device in accordance with the present disclosure;
FIG. 5 is a side cross-sectional view of an exemplary embodiment of a prosthetic device in accordance with the present disclosure;
FIG. 6A is a side cross-sectional view of an exemplary embodiment of a prosthetic device in accordance with the present disclosure;
FIG. 6B is a side cross-sectional view of an exemplary embodiment of a prosthetic device in accordance with the present disclosure;
FIG. 7 is a perspective view of an exemplary embodiment of a prosthetic device in accordance with the present disclosure;
FIG. 8 is a perspective view of an exemplary embodiment of a prosthetic device in accordance with the present disclosure;
FIG. 9 is a side cross-sectional view of an exemplary embodiment of a prosthetic device in accordance with the present disclosure; and
FIG. 10 is a side view of an exemplary embodiment of a prosthetic device in accordance with the present invention.

In the following detailed description of exemplary embodiments of the disclosure, reference is made to the accompanying drawings in which like references indicate similar elements, and in which is shown by way of illustration specific embodiments in which disclosed systems and devices may be practiced. These embodiments are described in sufficient detail to enable those skilled in the art to practice the embodiments, and it is to be understood that other embodiments may be utilized and that logical, mechanical, functional, and other changes may be made without departing from the scope of the present disclosure. The following detailed description is, therefore, not to be taken in a limiting sense, and the scope of the present disclosure is defined by the appended claims. As used in the present disclosure, the term "or" shall be understood to be defined as a logical disjunction and shall not indicate an exclusive disjunction.

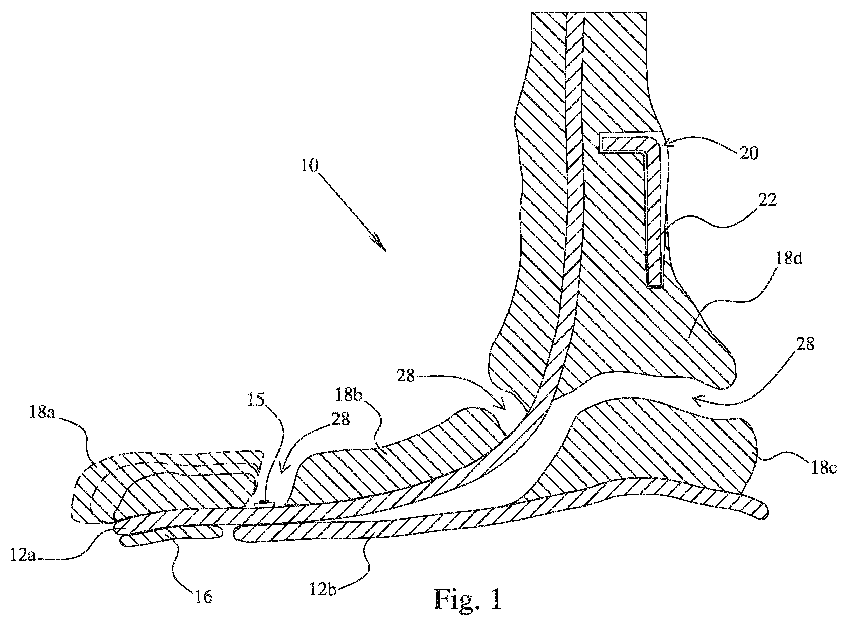

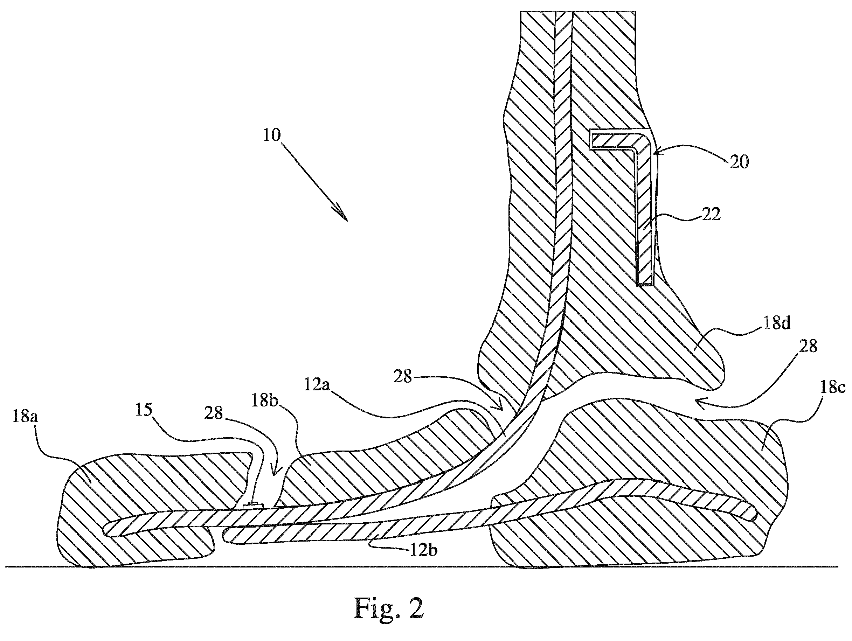

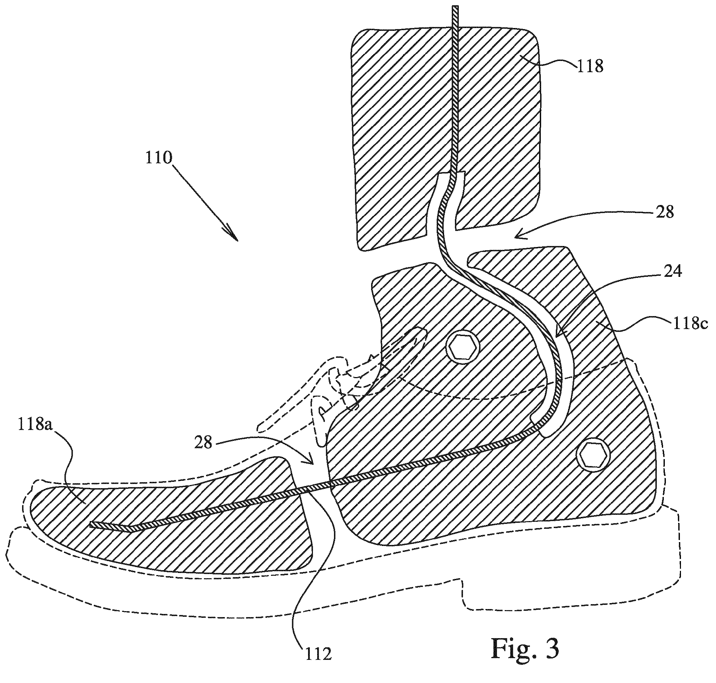

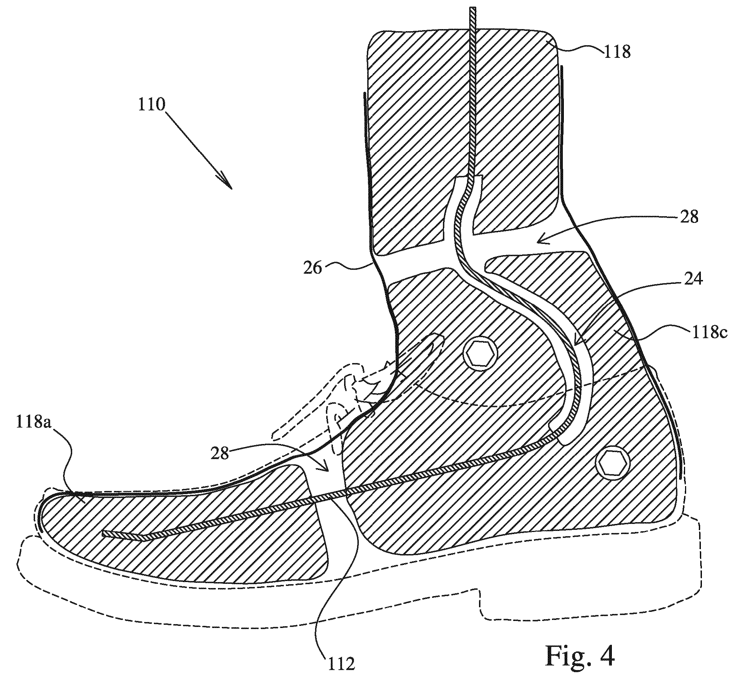

FIGS. 1 and 2 show exemplary embodiments of a prosthetic device 10. An exemplary prosthetic foot 10 comprises at least one leaf spring 12 and a plurality of polymer units 18 attached to the leaf spring. The prosthetic foot 10 may include a single leaf spring 112 (as shown in FIGS. 3 and 4), and in exemplary embodiments both a toe leaf spring 12a and a heel leaf spring 12b. The leaf springs 12 may be conventional fiber leaf springs or may be made of any other material that provides sufficient strength and flexibility. The toe leaf spring 12a may have a bend or curve and extend from the toe part of the prosthesis 10 past an ankle part and up to the top of the prosthesis. The toe and heel leaf springs 12a, 12b may be attached at one or more locations. In exemplary embodiments, the toe and heel leaf springs 12a, 12b are fastened together by one, two, or more bolts 15. In exemplary embodiments, the attachment point is at the top of the heel leaf spring 12b at the front end of the heel leaf spring 12b and at the bottom of the toe leaf spring 12a close to the front end of the toe leaf spring.

As best seen in FIG. 1, a toe pad 16 may be provided and attached to the bottom front of the toe leaf spring 12a. The toe pad 16 may be made of a tough pad material akin to material used in the soles of very high quality shoes. In exemplary embodiments, this material could be attached to the leaf spring 12 in segments. Such segments could be designed to maximize impact on leaf spring deflection, while providing the space filling role and the sizing role of a foot shell. Prosthetic foot designs could be adapted to a variety of leaf spring legs, in a kit format.

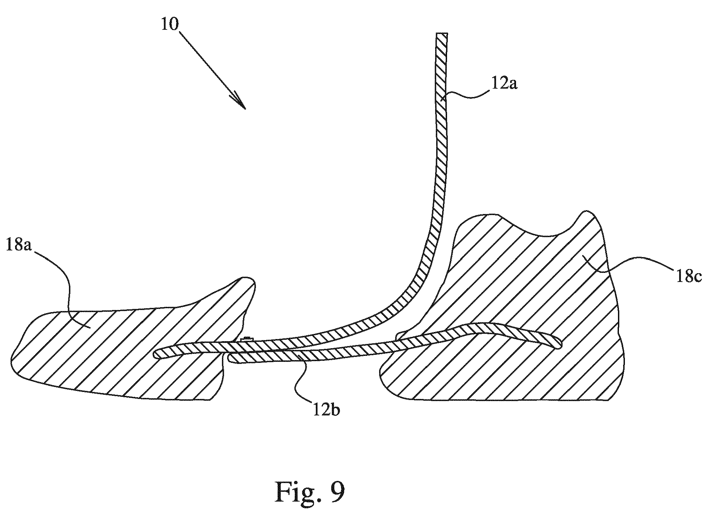

In exemplary embodiments, one or more pieces 18a-18d of polymer filler are attached to the top of the leaf springs. There will be minimal stress on these polymer units 18a-18d, as they are on top of the leaf springs 12, not on the ground/foot interface zone. The polymer units 18a-18d may comprise a heel piece 18c and a toe piece 18a. A relatively wide heel piece 18c and wide toe piece 18a can be used in combination with narrow leaf springs to provide a stable support platform. In exemplary embodiments, the prosthetic system 10 may include two, three, four, or more polymer units 18: a toe unit 18a, a middle or metatarsal unit 18b, one or more heel units 18c, and a shank unit 18d. As shown in FIG. 9, exemplary embodiments could include just two polymer units: a toe unit 18a and a heel unit 18c.

As shown in FIGS. 1 and 2, one of the polymer units 18 may define a storage compartment 20 therein. In exemplary embodiments, a polymer unit 18 includes a tool compartment 20 so the user can house a tool 22 needed to adjust or repair the device. More particularly, the shank polymer piece 18d can incorporate a tool/lubricant storage compartment 20 so that repairs and adjustments can be made in the field without an auxiliary tool kit.

As best seen in FIG. 2, one or both of the heel piece 18c and the toe piece 18a may extend far enough beneath the heel leaf spring 12b such that the leaf spring 12b does not touch the ground when the device 10 is used for ambulatory movement. This advantageously prevents damage to the leaf spring 12 in the event that gravel or other debris enters the shoe while the user is walking or running and becomes positioned under the spring. The toe piece 18a may extend further down than the heel piece 18c and may extend beneath the toe leaf spring 12a and/or heel leaf spring 12b such that extension of the toe piece 18a provides increased flexing of the leaf spring 12a. The extension of the polymer toe piece 18a beyond the toe end of the toe leaf spring 12a provides additional effective lever arm length that produces more flexing of the leaf spring 12a, thereby providing more perceived energy return at each toe lift. In exemplary embodiments, the polymer toe piece 18a can be modified to compress the toe sooner, thereby providing more energy return at "toe-off."

The toe piece 18a can be supplied in a variety of sizes so that one standard device 10 may be used for several shoe sizes by changing the toe piece only. Conveniently, toe piece 18a represents a variability option that will allow some sizing flexibility. In exemplary embodiments, it can cover two or three foot sizes. Advantageously, the interchangeable and replaceable polymer toe pieces 18a allow one size of leaf springs to be used for a range of shoe sizes, thereby reducing production costs. Moreover, it is typically cheaper to change polymer elements than to change the leaf spring. Worn or damaged polymer pieces may be replaced by removing the old pieces and attaching new pieces to the leaf springs 12.

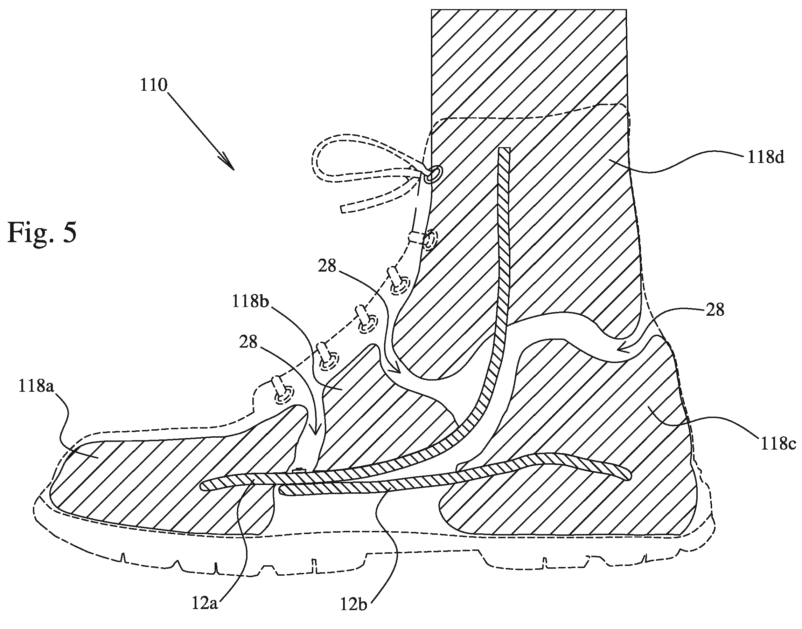

As shown in FIGS. 3, 4 and 5, in exemplary embodiments of the prosthetic device 110 the polymer units 118a, 118b, 118c, 118d are shaped so as to fill the interior of a conventional shoe, and may optionally be cosmetically shaped to resemble a foot. Advantageously, the cosmetic effect could be carried on up the leaf spring limb. A gaiter could be provided to achieve a snug fit and prevent water and debris from getting into the shoe or boot. The heel unit 118c could define a channel 24 to allow the leaf spring 12a, 12, 112 to flex. The prosthetic device 110 could also incorporate a split-shell design with attachment means such as bolts or other fasteners. As shown in FIG. 4, a cosmetic cover sock 26 could be provided to cover the device 110. More particularly, a sock 26 could be fashioned to attach to the sidewalls and the top of the prosthetic foot 110. A reinforced sock could be designed around the prosthetic system 110. Alternatively, a conventional sock can be worn over the prosthetic system 110. The toe piece 118a can provide stand-alone cosmetic effect, with no outer cosmetic covering required.

In exemplary embodiments, the polymer units 18, 118, 218a-d, 318, 418 are spaced apart and define spaces 28 between the units. The polymer pieces 18, 118, 218a-d, 318, 418 are spaced apart so they do not touch each other, or minimally touch, and do not interfere with the springs' 12, 112 flexibility. More particularly, the polymer units 18, 118, 218a-d, 318, 418 are advantageously spaced apart by spaces 28 so there is no contact between any two polymer units 18, 118, 218a-d, 318, 418 and the units do not touch each other, or minimally touch, even at the maximum deflection of the leaf springs 12, 112. In exemplary embodiments, there is a space 28 between the toe unit 18a, 118a, 218a and the middle or metatarsal unit 18b, 118b, 218b, a space 28 between the metatarsal unit 18b, 118b, 218b and the shank unit 18d, 118d, 218d, and a space 28 between the shank unit 18d, 118d, 218d and the heel unit 18c, 118c, 218c. The space 28 between the shank unit 18d, 118d, 218d and the heel unit 18c, 118c, 218c may extend downward, forming a space between a front surface of the heel unit 18c, 118c, 218c and the heel leaf spring 12b. As best seen in FIG. 4, in embodiments lacking a metatarsal unit, there could be a space 28 between the toe unit 118a and the heel unit 118c

To determine the sizes of the various spaces between polymer units, molds of prosthetic devices could be made for various foot sizes. A prosthetic device for each foot size would have spaces of appropriate size to prevent the polymer units from touching each other at the maximum deflection of the leaf springs. In this way, standard dimensions for each foot size could be developed. Although dimensions and space sizes would vary across the range of foot sizes, exemplary embodiments have a space 28 between the shank unit 18d, 118d, 218d and the heel unit 18c, 118c, 218c of between about 0.3 inches and 0.7 inches. In exemplary embodiments, the space between the heel unit 18c, 118c, 218c and the heel leaf spring 12b is between about 0.3 inches and 0.7 inches. To determine the space 28 between the toe unit 18a, 118a, 218a and the metatarsal unit 18b, 118b, 218b, one pertinent consideration is to maintain some clearance or distance between the back portion of the toe unit 18a, 118a, 218a and the bolt 15 holding the leaf springs together. Similarly, there should be some clearance or distance between the front of the metatarsal unit 18b, 118b, 218b and the bolt 15.

The spaces 28 advantageously prevent the polymer units 18, 118, 218a-d from squeezing together (compressing) and wasting energy when the leaf springs 12, 112 are flexed during walking and running. This also prevents rubbing noise. Without movement between the leaf spring limb 12, 112 and foam material of the polymer units 18, 18a-d, 118a-d, 218a-d the device 10, 110, 210 is quiet. Even when the user wears the prosthetic device 10, 110, 210 without a sock inside a variety of shoes or when the device gets wet, it is still quiet. The space between the polymer units 18, 18a-d, 118a-d, 218a-d allows for access/repair/replacement of the leaf spring toe piece 12a. It also enhances for replacement of any of the polymer units 18, 18a-d, 118a-d, 218a-d if they break down. Optionally, tape, fabric, soft foam, etc. could provide covering between the spaces by, for example, putting tape over the top or side walls of the device. This would not need to be done on the bottom of the foot.

In exemplary embodiments, the prosthetic system 10, 110, 210 is designed to eliminate any pockets that can trap water and the spaces 28 may facilitate free flow of liquids out of the device. Thus, another advantage of the spacing between polymer units 18, 18a-d, 118a-d, 218a-d is that water entering the shoe is free to drain and will not collect within the prosthetic device 10, 110, 210. In other words, water can get into the spaces 28 between the polymer units 18, 18a-d, 118a-d, 218a-d but cannot collect and build up and can freely flow through and out of the device in the event that the device becomes submerged during use.

The polymer units 18, 18a-d, 118a-d, 218a-d may be of various designs and materials. In exemplary embodiments, the units are made of a foam polymer. The polymer units 18, 18a-d, 118a-d, 218a-d could be made of urethane, polyurethane, any foam or rubber material, or any other material that can be used for shoe soles. In exemplary embodiments, each of the components (polymer pieces and leaf springs) is impermeable to water, so the device cannot absorb water. The polymer units could be solid to prevent water entry and improve durability. Alternatively, the units could be fully or partially hollow, and packed with variousmaterials, as a means by which to enhance lateral and medial stability.

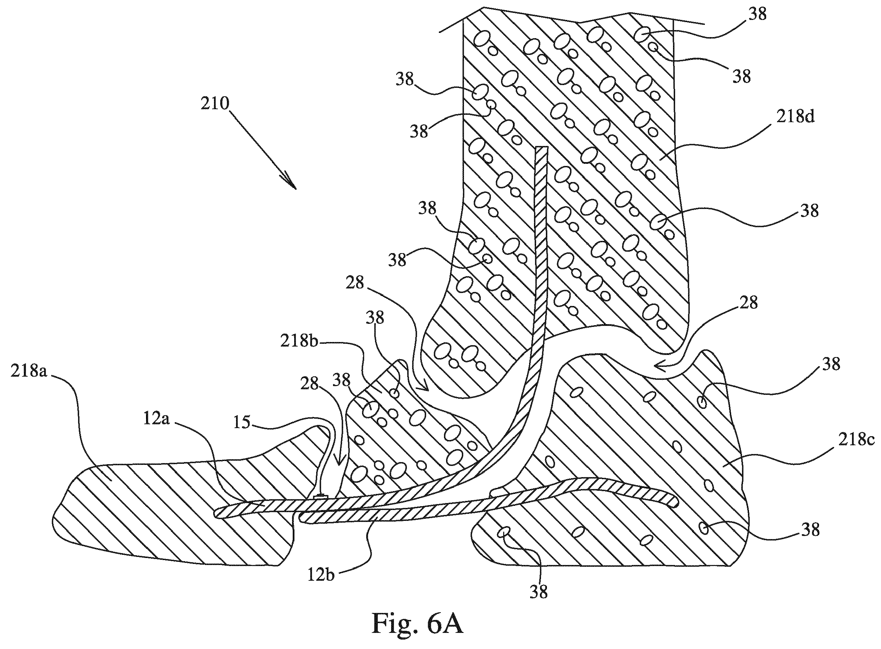

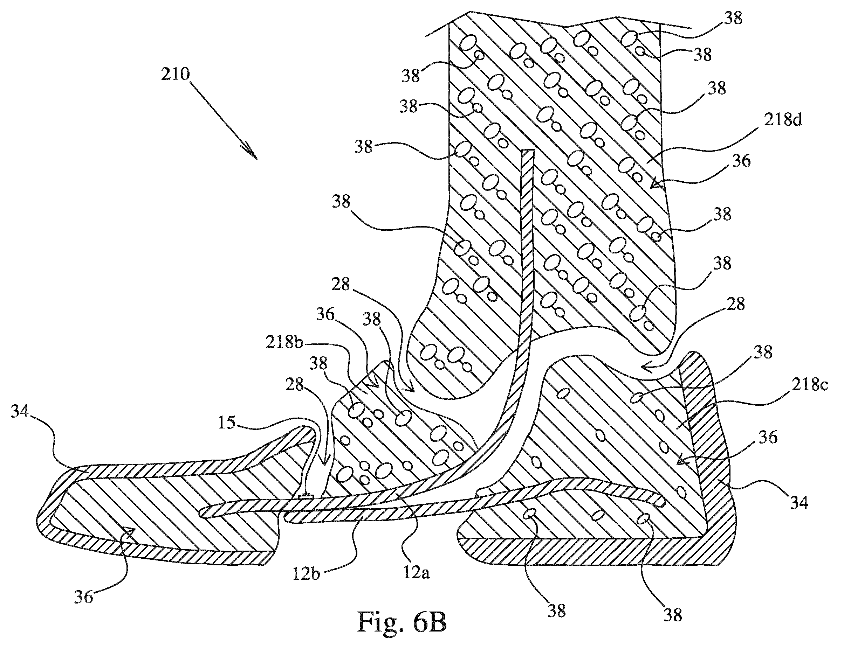

Advantageously, exemplary embodiments provide the ability to have site-specific durometers. Referring to FIGS. 6A and 6B, exemplary prosthetic devices 210 in which the polymer units 218a-218d vary in levels of hardness, or durometer, will now be described. The polymer heel 218c and toe pieces 218a can be manufactured with various values of stiffness, so that the device can be optimized for shock absorption and energy delivery to the springs for use by users of different body weights. For example, as shown in FIG. 6A, the durometer ranges between the heel piece 218c and toe piece 218a can be varied. In exemplary embodiments, the durometer of the toe piece 218a is higher than that of the heel piece 218c so the prosthetic device 210 is stiffer at the toe than at the heel to optimize performance and comfort. This is reflected by the lack of visible air bubbles or pores 38 in the toe piece 218a foam material and many pores 38 in the heel piece 218c.

A more rigid toe piece 218a and relatively softer heel piece 218c provide a number of advantages and may be desirable in certain applications. First, this design maximizes the energy return from the toe piece 218a such that the prosthetic device 210 provides better push-off. Also, a softer heel piece 218c dampens the heel strike or heel shock, providing more comfort for the user and making athletic activity much more bearable. Alternatively, there might be certain applications where a softer toe piece is also desirable. In exemplary embodiments, both the toe piece and the heel piece can be lower durometer and relatively soft, or the toe piece could be softer than the heel piece and the other polymer units. It should be noted that exemplary embodiments advantageously provide the ability to vary the relative durometer of all the polymer units in any number of permutations to provide different relative hardness and softness at various locations in the prosthetic device for different applications.

Different materials with various durometers can be provided for different settings. As discussed above, exemplary embodiments could have compressibility at the heel to dampen shock associated with heel strike (because the heel hits the ground first during use), and minimal to no compression at the toe to maximize for deflection of leaf spring and corresponding energy return at toe-off. As shown in FIG. 6A, the toe piece 218a could have a relatively high durometer (hard), with no air bubbles or pores 38, the middle piece 218b and shank piece 218d could have lower durometers and be softer with a large number of air bubbles 38, and the heel piece 218c could have an intermediate durometer or medium hardness with an intermediate number of air bubbles 38.

These variable durometer configurations could be optimized to enhance for performance and/or comfort. For example, the shank unit 218d could be made extremely compressible to maximize energy return at toe-off. A variety of durometers and durometer ranges could be provided. In exemplary embodiments, the durometer ranges for hard toe 218a and heel units 218c could be between about 30-90 on the Shore A scale, and in exemplary embodiments the durometer range is 40-80.

Exemplary embodiments of a heel piece 218c and/or toe piece 218a could have two or more different durometers in the same unit. For example, the heel piece 218c could have one durometer at the outer surface of the heel and a different durometer in the softer inner portion of the heel. As best seen in FIG. 6B, the polymer pieces 218a, 218c can be made with an outer shell 34 and an inner foam filling 36. More particularly, polymer units 18, 118, 218a-d could be packed with foam filling 36 and have an outer foam shell 34 so there is a durometer and/or density differential between the inner foam filling 36 and the outer foam shell 34 (with higher durometer represented by tighter diagonal lines and lower diameter represented by sparser diagonal lines).

In exemplary embodiments, the foam of the shell 34 is denser than the foam of the filling 36, has no visible pores 38, and has a durometer of about 40-100 on the Shore A scale. The outer foam shell 34 may be a relatively thin layer in the range of about 0.1 to about 0.5 inches. In addition, the inner portion of the polymer unit 218a, 218c could be packed with less foam material so it is partially hollow and has air bubbles or pores 38 in it. In such embodiments, there is space for the foam to rise or expand. In exemplary embodiments, there is less foam filling 36 in the inner part of the toe piece 218a or heel piece 218c.

In exemplary embodiments, the heel shell 34 durometer may be around 40-100 on the Shore A scale and the inner foam filling portion 36 could be between about 10 and about 30. Because the heel portion is in contact with moving parts more frequently than the softer ankle/calf portion, it is beneficial for the outer part of the heel to be a higher durometer so that the foam does not wear away too quickly. The inner foam is what the user would perceive as the softness of the heel. In exemplary embodiments, the soft portions of the foot may have a durometer in the range of about 10-30, but could vary based on the measurements of the foam heel bumpers and for different applications.

Due to the advantages of the foam polymer material, the heel and toe can be readily modified to optimize the point at which the leaf spring 12 starts to bend and store energy, providing a leverage, or cam, effect. This advantageously provides a new and compelling sense of energy return, or "bounce." The foam material doesn't just protect the leaf springs from rocks and grit, but due to its elevation inside the shoe (close to an inch in an exemplary embodiment), provides a comprehensive protective element for the leaf spring's toe and heel, whereas in a foot shell system this function requires another element typically bonded to the toe and heel leaf springs. Here the toe and heel foam units do double duty.

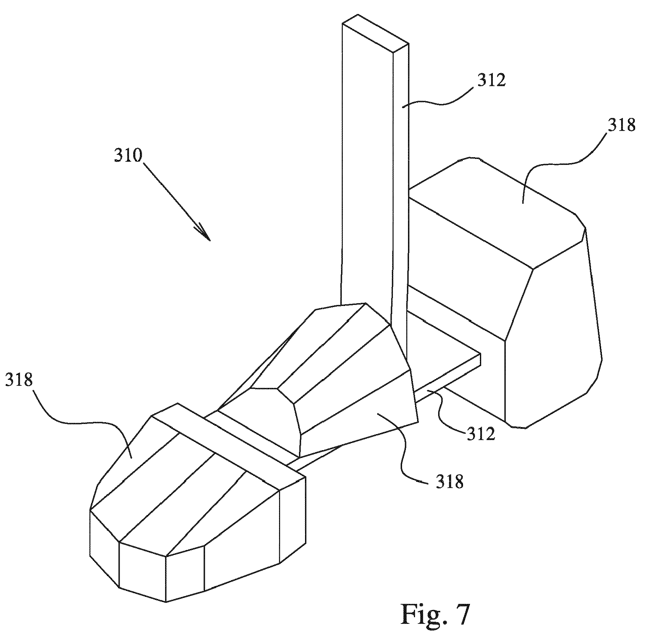

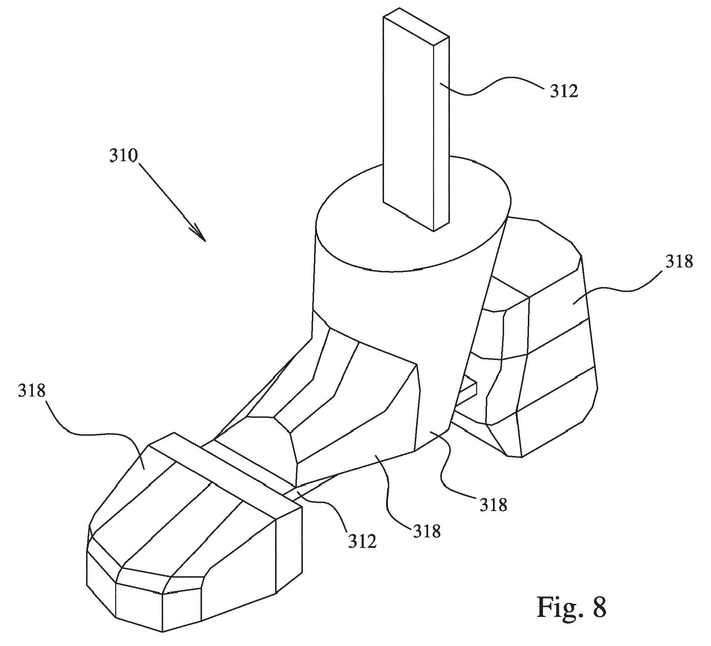

Referring to FIGS. 7 and 8, exemplary embodiments of a prosthetic device 310 could employ a more modern or futuristic design. In exemplary embodiments, the polymer units 318 attached to the leaf springs 312 could be angular components with square, rectangular, hexagonal, half-hexagonal, or other angular cross sections.

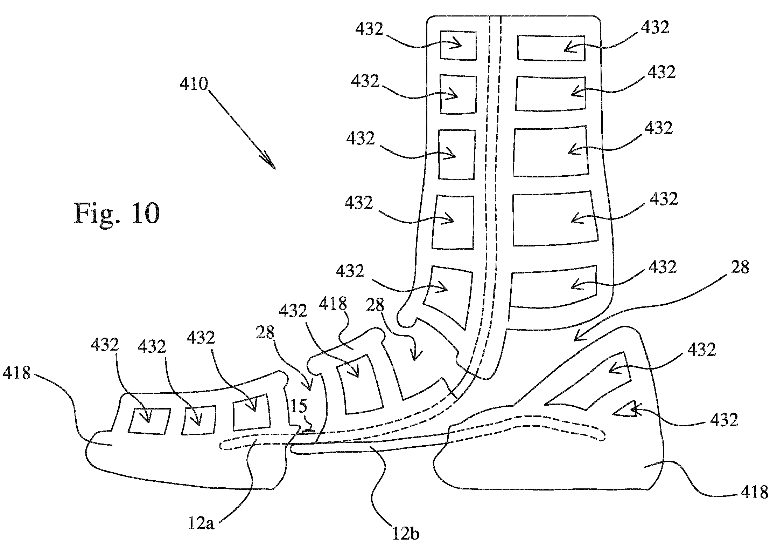

With reference to FIG. 10, exemplary embodiments of a prosthetic device 410 have a "sandal" configuration. Instead of each polymer unit 418 being solid or defining a single hollow space, one or more polymer units 418 may define a plurality of hollow portions 432 extending laterally through the units. The outer walls of the polymer units 418 surrounding the hollow portions 432 lend the appearance of a "sandal" design. Such sandal embodiments advantageously are lighter in weight, and the exo-skeletal form or effect makes it easier for the user to walk comfortably and appropriately in footwear. Moreover, sandal embodiments could be designed towards male and female appearance. Exemplary female sandal embodiments can include finer, more graceful or artistic "straps" and could be more slender and have more curvature.

While the disclosed systems and devices have been described in terms of what are presently considered to be the most practical exemplary embodiments, it is to be understood that the disclosure need not be limited to the disclosed embodiments. It is intended to cover various modifications and similar arrangements included within the spirit and scope of the claims, the scope of which should be accorded the broadest interpretation so as to encompass all such modifications and similar structures. The present disclosure includes any and all embodiments of the following claims.

Thus, it is seen that improved prosthetic devices and systems are provided. It should be understood that any of the foregoing configurations and specialized components or chemical compounds may be interchangeably used with any of the systems of the preceding embodiments. Although illustrative embodiments are described hereinabove, it will be evident to one skilled in the art that various changes and modifications may be made therein without departing from the disclosure. It is intended in the appended claims to cover all such changes and modifications that fall within the true spirit and scope of the disclosure.

## Claims

1. A prosthetic device (10, 110, 210, 310, 410) comprising:
at least one leaf spring (12, 12a, 12b, 112); and
a plurality of polymer units (18, 18a, 18b, 18c, 18d, 118a, 118b, 118c, 118d, 218a, 218b, 218c, 218d, 318) attached to the leaf spring, the polymer units being spaced apart and defining spaces (28) between the units such that contact between any two polymer units does not interfere with deflection of the one or more leaf springs.

2. The prosthetic device of claim 1 wherein the spaces (28) facilitate free flow of liquids out of the device.

3. The prosthetic device of claim 1 or claim 2, wherein the polymer units (18, 18a, 18b, 18c, 18d, 118a, 118c, 218a, 218b, 218c, 218d, 318) are impermeable to water.

4. The prosthetic device of claim any preceding claim, wherein the plurality of polymer units comprise a heel piece (18c, 118c, 218c) and a toe piece (18a, 118a, 218a).

5. The prosthetic device of claim 4, wherein one or both of the heel piece (18c, 118c, 218c) and the toe piece (18a, 118a, 218a) extend beneath the leaf spring (12, 12a, 12b, 112) such that the one or more leaf springs do not contact the ground when the device is used for ambulatory movement.

6. The prosthetic device of claim 4 or claim 5, wherein the toe piece (18a, 118a, 218a) extends further down than the heel piece (18c, 118c, 218c).

7. The prosthetic device of any preceding claim, wherein the plurality of polymer units comprise a toe piece (18a, 118a, 218a) extending beneath the one or more leaf springs (12, 12a, 12b, 112) such that extension of the toe piece provides increased flexing of the one or more leaf springs.

8. The prosthetic device of any preceding claim, wherein one of the plurality of polymer units (18d) defines a storage compartment (20) therein.

9. The prosthetic device of any preceding claim, wherein the polymer units vary in levels of hardness.

10. The prosthetic device of any preceding claim, wherein the plurality of polymer units includes a shank unit (18, 18d, 118d, 218d).

11. The prosthetic device of claim 9, wherein the plurality of polymer units comprise a heel piece (18c, 118c, 218c) and a toe piece (18a, 118a, 218a), the hardness of the toe piece being higher than the hardness of the heel piece, thereby providing higher energy return and better toe-off from the toe piece.

12. The prosthetic device of claim 11, wherein the polymer units further include a shank unit (18, 18d, 118d, 218d), the hardness of the shank unit being relatively lower than hardness of the heel piece (18c, 118c, 218c).

13. The prosthetic device of any preceding claim, wherein one or more polymer units (418) define a plurality of hollow portions (432).

14. The prosthetic device of any preceding claim, wherein at least one polymer unit (218a, 218c) comprises an outer shell (34) and an inner filling (36).

15. The prosthetic device of claim 14, wherein the outer shell (34) is denser than the inner filling (36).
